# FASCICULE DE BREVET EUROPEEN

(11) **EP 1 853 296 B1**
(45) Date de publication et mention de la délivrance du brevet: **20.06.2012**
(21) Numéro de dépôt: 06704161.6
(22) Date de dépôt: 10.01.2006
(51) Int. Cl.: A61K 38/13, A61P 9/10

(54) **UTILISATION D'UN UNDÉCAPEPTIDE CYCLIQUE POUR LA PRÉPARATION D'UN MÉDICAMENT ADMINISTRABLE LORS DE SITUATIONS ISCHÉMIQUES MYOCARDIAQUES**
VERWENDUNG EINES CYCLISCHEN UNDECAPEPTIDS ZUR HERSTELLUNG EINES MEDIKAMENTS ZUR VERABREICHUNG WÄHREND MYOKARDIALER ISCHÄMISCHER EREIGNISSE
USE OF A CYCLIC UNDECAPEPTIDE FOR THE PREPARATION OF A MEDICAMENT FOR ADMINISTRATION DURING MYOCARDIAL ISCHAEMIC EVENTS

(30) Priorité: 10.01.2005 EP 05000357
(43) Date de publication de la demande: 14.11.2007
(73) Titulaire: DEBIOPHARM S.A., 1006 Lausanne (CH)
(72) Inventeur: SCALFARO, Pietro, CH-1005 Lausanne (CH); DUMONT, Jean-Maurice, CH-1009 Pully (CH); VUAGNIAUX, Grégoire, CH-1005 Lausanne (CH)
(74) Mandataire: Grosfillier, Philippe
(86) Numéro de dépôt international: PCT/EP2006/050140
(87) Numéro de publication internationale: WO 2006/072639

(56) Documents cités:
- WO-A-00/01715
- US-B1- 6 790 935
- DI LISA FABIO ET AL: "Opening of the mitochondrial permeability transition pore causes depletion of mitochondrial and cytosolic NAD+ and is a causative event in the death of myocytes in postischemic reperfusion of the heart" JOURNAL OF BIOLOGICAL CHEMISTRY, vol. 276, no. 4, 26 janvier 2001 (2001-01-26), pages 2571-2575, XP002330700 ISSN: 0021-9258
- HANSSON M J ET AL: "The nonimmunosuppressive cyclosporin analogs NIM811 and UNIL025 display nanomolar potencies on permeability transition in brain-derived mitochondria" JOURNAL OF BIOENERGETICS AND BIOMEMBRANES, PLENUM PUBLISHING, NEW YORK, NY, US, vol. 36, no. 4, août 2004 (2004-08), pages 407-413, XP002330699 ISSN: 0145-479X
- NIEMANN CLAUS U ET AL: "Close association between the reduction in myocardial energy metabolism and infarct size: Dose-response assessment of cyclosporine" JOURNAL OF PHARMACOLOGY AND EXPERIMENTAL THERAPEUTICS, vol. 302, no. 3, septembre 2002 (2002-09), pages 1123-1128, XP002381017 ISSN: 0022-3565
- HAUSENLOY DEREK J ET AL: "Inhibiting mitochondrial permeability transition pore opening: A new paradigm for myocardial preconditioning?" CARDIOVASCULAR RESEARCH, vol. 55, no. 3, 15 août 2002 (2002-08-15), pages 534-543, XP002381018 ISSN: 0008-6363

## Description

La présente invention concerne l'utilisation d'un undécapeptide cyclique pour la préparation d'un médicament administrable lors d'une situation d'ischémie myocardique.

Les ischémies myocardiques sont définies comme un déséquilibre entre besoins et apports en oxygène. Ce déséquilibre conduit à un désordre de la fonction cardiaque. Les ischémies myocardiques sont, dans la grande majorité des cas, causées par une insuffisance de la circulation sanguine vers le tissu musculaire cardiaque, privant ou diminuant drastiquement ainsi les cellules myocardiques en apport d'oxygène. Ces ischémies peuvent être dues à l'obstruction d'un vaisseau (thrombose) ou à la réduction du diamètre interne d'une artère (sténose) ou à une diminution du flux sanguin coronarien (hypoperfusion) tel que dans les états d'insuffisance circulatoire lors d'un sepsis sévère avec choc endotoxinémique. En ce qui concerne le sepsis sévère, il entraîne également un dysfonctionnement hémodynamique avec une dépression myocardique directe.

La reperfusion se définit comme le rétablissement d'une circulation sanguine adéquate au sein d'un tissu ischémique permettant d'atteindre à nouveau l'équilibre entre besoins et apports en oxygène. La reperfusion lors d'interruption complète du flux sanguin coronarien est effectuée par désobstruction de l'artère occluse.

Les infarctus sont consécutifs à des ischémies. Le terme d'infarctus définit un foyer circonscrit de nécrose tissulaire. Ainsi, l'infarctus du myocarde entraîne la destruction d'une partie du coeur du fait de la mort des cellules du muscle cardiaque.

L'infarctus du myocarde est une maladie malheureusement très fréquente. A titre d'exemple, en France, environ 180'000 à 200'000 personnes par an sont affectés par cette maladie prédominante chez l'homme. Elle apparaît, en priorité, chez les sujets ayant des facteurs de risques cardiovasculaires tels que la consommation de tabac, l'obésité, un diabète, l'hyperlipidémie ou une hypertension artérielle.

Lors de sepsis sévère une hypoperfusion myocyardique est accompagnée d'une dépression myocardique directe. A l'heure actuelle, il n'est pas clair quel mécanisme est prédominant (hypoperfusion ou dépression myocardique par des cytokines circulants) conduisant à une diminution de la fonction du myocarde. Néanmoins, il est bien connu que le patient avec sepsis sévère souffre souvent d'une défaillance cardiovasculaire avec dysfonctionnement myocardique.

L'infarctus aigu du myocarde ("Acute Myocardial Infarction", "AMI") constitue une urgence cardiologique absolue qui implique une prise en charge par des services médicaux et hospitaliers spécialisés avec un traitement de la phase aiguë ayant pour but de reperfuser le muscle cardiaque ischémique et de prévenir et/ou limiter les complications possibles liées à l'infarctus entraînant fréquemment le décès des patients dans les premières heures ou les premiers jours.

L'étendue de l'infarctus du myocarde est un élément déterminant pour la récupération fonctionnelle contractile du myocarde et du pronostic à long terme des patients.

Bien que la reperfusion protège incontestablement des cellules myocardiques de la mort cellulaire causée par la persistance de l'ischémie, elle s'accompagne aussi d'effets délétères sur la fonction contractile (sidération myocardique), le rythme cardiaque (survenue d'arythmies) et la perfusion tissulaire ("no-reflow"). Des données récentes indiquent même que la reperfusion peut aussi paradoxalement tuer une partie des cellules reperfusées (nécrose de reperfusion).

Lors de la reperfusion de l'infarctus du myocarde, des médicaments appartenant à des classes thérapeutiques différentes telles que, par exemple, celles des inhibiteurs de l'agrégation plaquettaire comme l'acide acétylsalicylique, celle des béta-bloquants, celle des inhibiteurs de l'enzyme de conversion (IEC) ou celle des statines ont un apport bénéfique sur le pronostic des patients. Toutefois, aucun de ces médicaments ou d'autres médicaments disponibles actuellement administrés lors de la reperfusion n'est pas capable de limiter la taille de l'infarctus du myocarde.

En revanche, la répétition d'épisodes d'ischémie-reperfusion de durée brève (ne provoquant pas par eux-mêmes de lésion cellulaire irréversible) réalisée soit avant ("pré-conditionnement"), soit après ("post-conditionnement") la période d'ischémie responsable de l'infarctus, ont montré *in vivo* un effet protecteur endogène et une limitation de la taille de l'infarctus. Cependant, les thérapies de pré-conditionnement ne sont pas applicables en clinique aux patients AMI pour des raisons physiopathologiques et pratiques. Quant aux traitements de post-conditionnement, ils ne s'appliquent qu'à un nombre limité de patients présentant un infarctus aigu du myocarde, comme ceux pouvant être traités par angioplastie coronaire transluminale percutanée.

Bien que les mécanismes cellulaires et enzymatiques impliqués dans les lésions nécrotiques du myocarde dues à une ischémie-reperfusion ne soient pas complètement élucidés, Di Lisa et al. sont d'avis, dans J. Biol. Chem., 2001, 276(4), 2571-2575, que l'ouverture de pores situés dans la membrane interne de la mitochondrie, appelés pores de transition de perméabilité mitochondriale ("Mitochondrial Permeability Transition Pore", MPTP) joue un rôle prépondérant dans la mort des cellules du myocarde lors de reperfusion après ischémie. Ainsi, une action inhibitrice directe ou indirecte de l'ouverture de ces MPTP par un médicament tel que la Cyclosporine A conduit à une diminution de l'effet délétère des phénomènes induits par une ischémie-reperfusion sur la viabilité du tissu cardiaque.

Cependant deux points suggèrent qu'une simple inhibition du MPTP, bien que nécessaire, n'est pas suffisante pour obtenir un effet protecteur du myocarde dans les situations d'ischémie-reperfusion susmentionnées. D'une part, il existe des inhibiteurs du MPTP comme le glutathion, qui présente un effet additif avec la Cyclosporine A sur l'inhibition du MTPT. Cependant, une protection des phénomènes induits par l'ischémie-reperfusion n'est néanmoins pas obtenue ou alors uniquement si le traitement est associé à d'autres facteurs protecteurs tel l'entraînement de la fonction contractile du myocarde. D'autre part, la chronologie d'administration proposée par différents auteurs dans les modèles expérimentaux est questionnable pour obtenir des effets bénéfiques induits par l'inhibition du MPTP. L'administration proposée dans beaucoup de modèles expérimentaux correspond à une perfusion continuelle du coeur avant, pendant et après la période d'ischémie, se poursuivant pendant toute ou partie de la phase de reperfusion. Cet aspect d'administration n'est pas applicable tel quel en clinique et le mode d'administration optimal n'est pas connu.

Ainsi, de nouvelles thérapies adjuvantes qui doivent être administrées pendant la reperfusion, qui ont pour effet cardioprotecteur de limiter la taille de la nécrose du myocarde et d'améliorer sa fonction, sont nécessaires dans le traitement de certaines situations d'ischémie-reperfusion myocardique, en particulier lors d'un infarctus aigu du myocarde et/ou d'une dépression et dysfonctionnement du myocarde dus à un sepsis sévère.

De plus, la fenêtre de temps pendant laquelle le médicament est administré est importante. L'administration du médicament doit débuter impérativement avant la reperfusion, et se poursuivre éventuellement au cours de celle-ci, afin d'obtenir le bénéfice escompté en terme de limitation de la taille de l'infarctus du myocarde, de la récupération contractile musculaire et de la survie du patient.

Le but de la présente invention est de mettre à la disposition du clinicien une médication lui permettant de traiter certaines situations d'ischémies myocardiques, en particulier lors d'un infarctus aigu et/ou suite à un sepsis sévère, dans le traitement adjuvant à la reperfusion myocardique et qui a pour effet de limiter la taille de l'infarctus et d'améliorer sa fonction.

Il a été trouvé, de manière très surprenante, que ce but peut être atteint par l'utilisation d'un undécapeptide cyclique particulier.

Ainsi, la présente invention a pour objet l'utilisation d'un undécapeptide cyclique ayant la structure de formule (I) 
-MeBmt-αAbu-MeAla-EtVal-Val-MeLeu-Ala-(D)Ala-MeLeu-MeLeu-MeVal-
1 2 3 4 5 6 7 8 9 10 11
Formule (I)
 pour la préparation d'un médicament administrable lors d'une situation d'ischémie myocardique et devant être administré préalablement et/ou pendant le rétablissement dudit flux sanguin.

La structure chimique du undécapeptide cyclique de formule (I) ayant un certain nombre de points communs avec celle de la Cyclosporine A, sa formule est donnée en utilisant la nomenclature habituellement utilisée pour caractériser cette Cyclosporine A. Selon cette nomenclature, MeBmt est l'abréviation de l'acide aminé N-méthyl-(4R)-4-but-2E-èn-1-yl-4-méthyl -(L)thréonine, αAbu celle de l'acide L-α-aminobutyrique, MeAla celle de la N-méthyl-L-alanine, EtVal celle de la N-éthyl-L-valine, Val celle de la valine, MeLeu celle de la N-méthyl-L-Leucine, Ala celle de la L-alanine, (D)Ala celle de la D-alanine et MeVal celle de la N-méthyl-L-valine. La numérotation habituellement utilisée pour caractériser la position respective de chacun des acides aminés constituant la Cyclosporine A est également utilisée pour définir la structure du undécapeptide cyclique de formule (I). Toujours en référence à la Cyclosporine A, l'undécapeptide cyclique de formule (I) peut être également appelé [MeAla]³-[EtVal]⁴-CsA.

L'undécapeptide cyclique ayant la structure de formule (I) et sa préparation ont été décrits par J.F. Guichoux dans "De nouveaux analogues de Cyclosporin A comme agent anti-HIV-1", Thèse de doctorat, Faculté des Sciences de l'Université de Lausanne, 2002, et par R. Wenger et al. dans la demande de brevet internationale WO 00/01715 et le numéro de registre qui lui a été attribué par Chemical Abstracts Service est CAS RN 254435-95-5.

Ce produit, ainsi que certains de ses analogues structuraux, sont présentés dans ces publications comme ayant une forte activité pour inhiber l'action du virus de l'immunodéficience humaine, agent viral responsable du SIDA, tout en ne présentant pas les propriétés immunosuppressives bien connues de la Cyclosporine A. De plus, un potentiel neuroprotecteur lui est aussi reconnu, en particulier l'inhibition du MPTP de mitochondries isolées du cerveau, par Hansson et al. dans J. Bioenerg. Biomembr., 2004, 36, 407-413.

Les situations d'ischémies myocardiques, au cours desquelles le undécapeptide cyclique de formule (I) peut être administré, résultent de la diminution ou de l'arrêt de la circulation sanguine myocardique, ayant pour conséquence, entre autre, une réduction ou l'arrêt de l'apport d'oxygène vers cet organe nécessitant un rétablissement du flux sanguin ou une reperfusion cardiaque. Ces situations d'ischémie peuvent se manifester par des dysfonctionnements cardiaques détectables entre autre par écho- et électrocardiographie. Ce phénomène conduit à des lésions cellulaires cardiaques détectables entre autre par mesures des marqueurs biologiques tels que les enzymes cardiaque, l'imagerie médicale telle que échographie, scintigraphie nucléaire, ou résonance magnétique nucléaire.

Une ischémie myocardique aiguë, sub-aiguë ou chronique peut être provoquée par, ou être associée à, une variété de facteurs ou maladies. Parmi ces facteurs ou maladies, on peut citer à titre d'exemple l'infarctus aigu du myocarde, l'angine de poitrine, l'angor instable, les maladies athéromateuses avec thromboembolie, les vasospasmes, les anévrysmes des petites et moyennes artères et des grands vaisseaux, l'hypotension artérielle due à une maladie du coeur ou systémique incluant des infections graves tel un sepsis sévère avec ou sans choc septique ou des réactions allergiques, une hypotension due à l'effet de un ou plusieurs médicaments, drogues, poisons, produits toxiques.

De plus, on peut également citer à titre d'exemple les hypoperfusions secondaires faisant suite à l'une ou l'autre des maladies ou situations suivantes: diabète sucré, hyperlipidémie, thromboangéite oblitérante ou maladie de Buerger, syndrome de Takayasu, syphilis cardiovasculaire, désordre des tissus conjonctifs tels que la maladie de Raynaud, phlegmatia caerulea dolens ou phlébite bleue de Grégoire, traumatisme des vaisseaux sanguins incluant les traumatismes iatrogéniques tels que des actes de chirurgie ou de transplantations d'organe, opérations du coeur et des grands vaisseaux avec ou sans recours aux techniques de circulation extracorporelle. Ces actes incluent également à titre d'exemple l'insertion chirurgicale d'implants, de dispositifs, de greffes, de prothèses ou tout autre dispositif ou produit biomédical, en particulier cardiologiques. Une liste non-limitative des organes ou tissus dans lesquels peuvent se présenter ces situations ischémiques comprend le coeur, le cerveau, les reins, les extrémités, la rate, le foie, l'estomac et le système gastro-intestinale incluant le petit intestin, le colon et le rectum, les poumons et les voies respiratoires, les yeux, la peau, les muscles, le pancréas, la prostate, la moelle osseuse, les glandes endocrines.

L'utilisation du undécapeptide cyclique de formule (I) est le traitement de situations d'ischémie myocardique, pour le traitement d'une hypoperfusion-reperfusion myocardique, en particulier lors de la survenue d'un infarctus aigu du myocarde et/ou de dysfonctionnement cardiaque lors de sepsis sévère.

Lors de l'utilisation du undécapeptide cyclique de formule (I), le traitement des situations liées aux ischémique myocardiques consiste à administrer au sujet ledit undécapeptide cyclique préalablement et/ou pendant le rétablissement du flux sanguin ou à une perfusion en adéquation avec les besoins en oxygène dans le coeur ou le tissu cardiaque. Ainsi, la circulation sanguine est rétablie par une reperfusion obtenue mécaniquement par angioplastie coronaire et/ou médicalement à la suite d'un traitement de fibrinolyse et/ou à la suite de mesures de réanimation médicamenteuse. L'undécapeptide cyclique de formule (I) est administré de préférence préalablement à ladite reperfusion. Dans ce cas, le début de l'administration s'échelonne au cours d'une période allant, de préférence, de la trentième minute précédant la reperfusion, de préférence encore au cours des 10 minutes précédant cette reperfusion, de préférence encore au cours des 5 minutes précédant cette reperfusion, de préférence encore au cours de la minute précédant cette reperfusion, jusqu'au moment de la réouverture de l'artère et/ou depuis le rétablissement du flux sanguin.

La préparation pharmaceutique comprenant le dérivé est administrée par voie intraveineuse, intra-artérielle, intra-coronarienne, ou intra-myocardique de préférence en bolus et/ou suivi en perfusion continue pendant les dix heures qui suivent le début de la reperfusion. L'administration de la préparation pharmaceutique en perfusion continue peut alors se faire par voie intra-veineuse, ou par voie intra-artérielle.

Lorsque le rétablissement du flux sanguin est initié par voie médicamenteuse, l'administration du undécapeptide cyclique de formule (I) peut se faire séparément et/ou préalablement ou de façon concomitante à cette administration médicamenteuse et peut se poursuivre au cours de cette administration médicamenteuse puis pendant les dix heures qui suivent le rétablissement du flux sanguin. La préparation pharmaceutique comprenant l'undécapeptide cyclique de formule (I) peut alors être administrée en dose unique par voie intra-veineuse, de préférence en bolus, puis elle peut se poursuivre en perfusion continue pendant les dix heures qui suivent le début de la reperfusion.

La composition pharmaceutique comprenant l'undécapeptide cyclique de formule (I) comme composé actif se présente sous forme de solution, de dispersion, ou sous forme de formulations de dépôts injectables. Ces formulations peuvent comprendre le composé actif sous forme de nano-cristaux, de micelles, d'émulsions lipidiques, de microémulsions ou de suspensions nanoparticulaires. Les compositions pharmaceutiques pour solutions injectables comprennent ledit undécapeptide en combinaison avec au moins un véhicule pharmaceutique acceptable. Avant l'administration, les compositions concentrées sont combinées avec des diluants appropriés, comprenant au moins un excipient, tel que un agent isotonique, un tampon ou un autre agent contrôlant le pH, et un conservateur. Ces excipients peuvent être ajoutés pour le maintien de la composition, d'un intervalle de pH d'environ 5.5 à environ 8.5 et de l'osmolarité d'environ 280 à environ 400 mosm/L. De manière générale, les formulations habituellement utilisées pour la préparation d'un médicament à base de Cyclosporine A sont également appropriées pour mettre en oeuvre la présente invention.

L'utilisation du undécapeptide cyclique de formule (I) peut également se faire en complément d'un traitement anti-angineux standard (bêta-bloquant, dérivé nitré retard, antagoniste calcique, antiplaquettaire) et/ou d'un traitement du choc septique. L'administration du undécapeptide est alors soit concomitante ou soit séquentielle avec au moins un second composé actif dans lesdits traitements.

De préférence, lors du traitement d'une ischémie-reperfusion myocardique, l'undécapeptide cyclique de formule (I) est administré à une dose allant de 0,1 mg/kg à 30 mg/kg, de préférence de 0,1 mg/kg à 20 mg/kg.

Le spécialiste sait établir la modalité d'administration, tant en ce qui concerne la chronologie, le mode d'administration ou la dose, de sorte qu'une concentration efficace du undécapeptide cyclique atteigne le tissu cardiaque hypoperfusé dès les premières secondes de la reperfusion.

L'invention et tous ses effets seront expliqués en détail à l'aide du dessin et des exemples.

Dans le dessin:

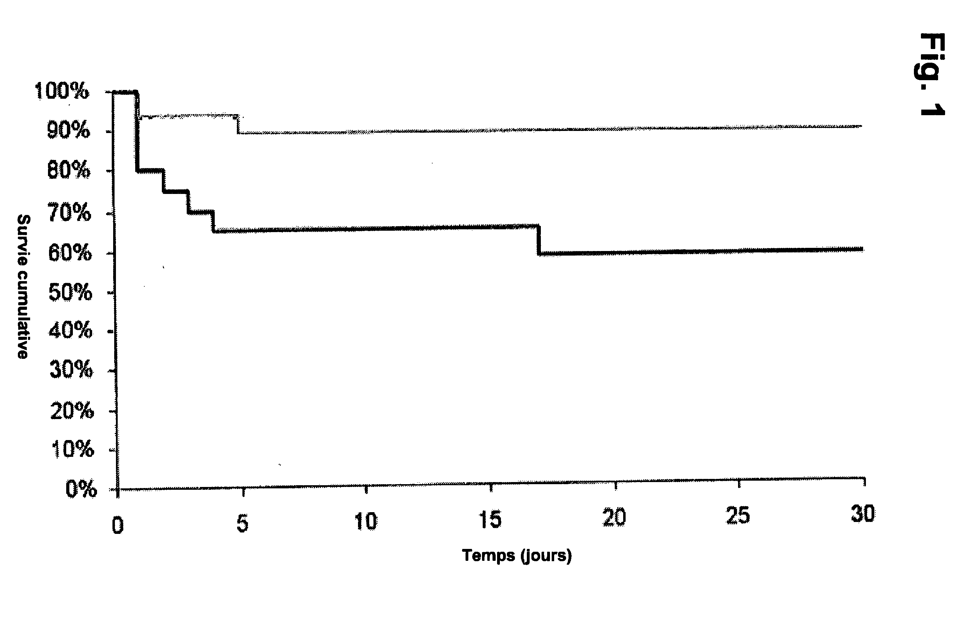

- la Fig. 1 représente les courbes de survie d'un modèle d'ischémie-reperfusion chez la souris et l'influence du undécapeptide cyclique de formule (I) sur la période de post-infarctus. La courbe noire représente le taux de survie au cours du temps des souris traitées avec le véhicule pharmaceutique seul après reperfusion et la courbe claire des souris traitées avec le undécapeptide de l'invention; et

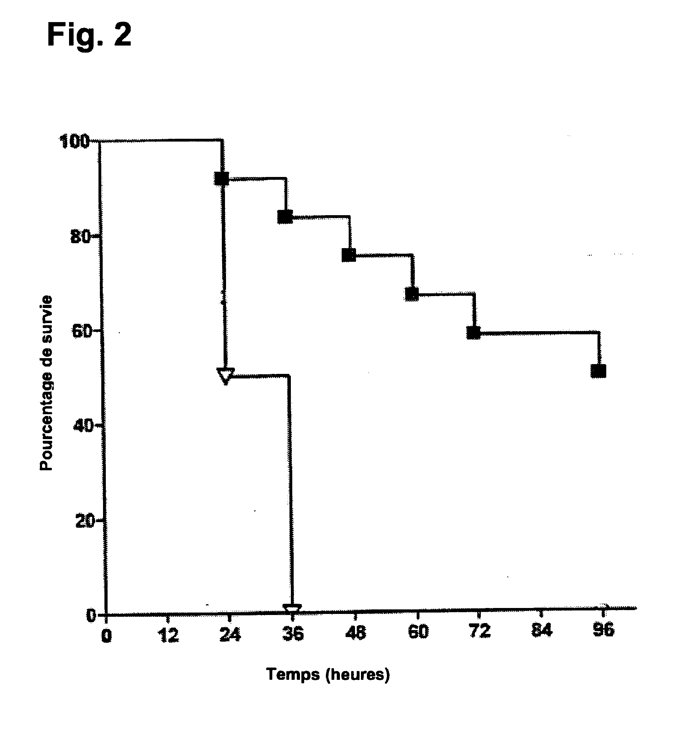

- la Fig. 2 représente les courbes de survie d'un modèle de choc septique chez la souris et l'influence du undécapeptique cyclique de formule (I) sur la période post-septique. La courbe avec les triangles représente le taux de survie au cours du temps des souris traitées avec du sérum physiologique uniquement après induction du choc septique et la courbe avec les carrés, les souris traitées avec le undécapeptide de l'invention.

Au cours des exemples qui suivent, la formulation suivante a été utilisée:

| | |
|---|---|
| undécapeptide cyclique de formule (I) | 35 mg/ml |
| cremophor EL | 650 mg/ml, |
| éthanol | 261 mg/ml |

### Exemple 1: Modèle d'ischémie-réperfusion chez le lapin - Effet cardioprotecteur du undécapeptide cyclique de formule (I)

Le but de cette étude est de tester l'effet cardioprotecteur du undécapeptide cyclique de formule (I), autrement appelé [MeAla]³-[EtVal]⁴-CsA, en terme de réduction de la taille de l'infarctus du myocarde mesuré 4 heures après la survenue de l'infarctus.

Après trachéotomie et ventilation par l'air ambiant, une thorocatomie est réalisée dans le quatrième espace intercostal gauche. Une branche marginale de l'artère coronaire circonflexe gauche est ligaturée à l'aide d'un fil de soie à suture 3.0. Pendant l'intervention, la température, la fréquence cardiaque et la pression sanguine sont contrôlée. Après 30 minutes d'occlusion, la ligature est levée (reperfusion) et les animaux sont maintenus pendant 4 heures dans une atmosphère contrôlée, avant euthanasie.

Un groupe d'animaux reçoit le véhicule pharmaceutique de [MeAla]3-[EtVal]4-CsA, un autre groupe est traité par [MeAla]3-[EtVal]4-CsA par voie intraveineuse sous forme d'injection unique respectivement de 20 mg/kg, une minute avant la levée de la ligature. Quatre heures après la reperfusion, l'artère coronaire est brièvement ré-occluse et un colorant bleu est injecté par voie intraveineuse de façon à déterminer la zone à risque. Les animaux sont anesthésiés et le coeur excisé est coupé en 5 à 6 fines tranches qui sont pesées. La surface basale de ces tranches est photographiée. L'incubation de ces tranches dans du 2,3,5-triphenyltetrazolium pendant 5 minutes à 37°C permet de différencier les zones du myocarde vivantes (rouge brique) et nécrosées (jaune pâle). Les tranches sont photographiées. Les aires des zones à risque et du myocarde infarci ou aire de nécroses sont déterminées par planimétrie. La taille de l'infarctus et de la zone à risque sont calculées et exprimées en pourcentage du poids du ventricule gauche.

**Tableau 1: Taille de l'infarctus du myocarde déterminée par le rapport des aires de nécroses (AN) sur les aires des zones à risques (AR) en pourcentage. SD représente la déviation standard.**

| Composé | Nombre d'animaux par groupe | Taille de l'infarctus (AN/AR ± SD) [%] |
|---|---|---|
| Véhicule pharmaceutique | 10 | 49 ± 9.5 |
| [MeAla]3-[EtVal]4-CsA | 8 | 29 ± 6.0 |

Il a été constaté que l'utilisation de [MeAla]3-[EtVal]4-CsA permet d'obtenir une diminution de la taille de l'infarctus dans la zone à risque de l'ordre de 40% par rapport aux animaux n'ayant reçu que le véhicule pharmaceutique.

### Exemple 2: Modèle d'ischémie-reperfusion chez la souris - Influence de [MeAla]3-[EtVal]4-CsA sur la période post-infarctus (remodelage ventriculaire et survie)

Après anesthésie, les souris sont intubées et ventilées par un ventilateur à rongeur. La température rectale est contrôlée et maintenue à 38°-39°C. Après sternotomie, l'artère interventriculaire antérieure est ligaturée par un fil en polypropylène 8-0. L'ischémie est visualisée par l'apparition de l'élévation du segment ST sur l'ECG (électrocardiogramme) et la pâleur du myocarde. Après 25 minutes d'occlusion, la ligature est levée et la reperfusion contrôlée par inspection visuelle et disparition du sus-décalage du segment ST. La paroi thoracique est fermée par suture et les souris maintenues dans une atmosphère à température contrôlée.

Un groupe d'animaux reçoit le véhicule de [MeAla]3-[EtVal]4-CsA; un autre groupe est traité par [MeAla]3-[EtVal]4-CsA par voie intraveineuse (injection unique à 10 mg/kg), 3 minutes avant la levée de la ligature. Les animaux sont suivis pendant 30 jours et la mortalité rapportée pour chaque groupe.

Quatre semaines après la reperfusion, le remodelage du ventricule gauche est étudié par échocardiographie-Doppler et la fraction d'éjection du ventricule gauche est mesurée.

Dans un groupe d'animaux ayant subi 25 minutes d'occlusion coronaire suivies de 24 heures de reperfusion, la zone à risque et la taille de l'infarctus sont déterminées. Après une brève ré-occlusion de l'interventriculaire antérieure, un colorant bleu est injecté via la veine cave de façon à déterminer la zone à risque. Les animaux sont anesthésiés et le coeur excisé. Le ventricule est coupé en 5 fines tranches et photographié. L'incubation de ces tranches dans du 2,3,5-triphenyltetrazolium pendant 15 minutes à 37°C permet de différencier les zones du myocarde vivantes (rouge brique) et nécrosées (jaune pâle). Les tranches sont photographiées. Les aires des zones à risque et du myocarde infarci sont déterminées par planimétrie. La taille de l'infarctus et de la zone à risque sont calculées et exprimées en pourcentage du poids du ventricule gauche.

**Tableau 2: Taille de l'infarctus du myocarde déterminée par le rapport des aires de nécroses (AN) sur les aires des zones à risques (AR) en pourcentage 24 heure après reperfusion. SD représente la déviation standard.**

| Composé | Nombre d'animaux par groupe | Taille de l'infarctus (AN/AR ± SD) [%] |
|---|---|---|
| Véhicule pharmaceutique | 10 | 61.4 ± 5.8 |
| [MeAla]3-[EtVal]4-CsA | 7 | 32.1 ± 7.3 |

**Tableau 3: Remodelage du ventricule gauche étudié par échocardiographie-Doppler: fraction d'éjection du ventricule gauche 4 semaines après la reperfusion. SD représente la déviation standard.**

| Composé | Nombre d'animaux par groupe | Fraction d'éjection du ventricule gauche ± SD [%] |
|---|---|---|
| Véhicule pharmaceutique | 10 | 62 ± 12 |
| [MeAla]3-[EtVal]4-CsA | 14 | 77 ± 6 |

Il a été constaté que l'utilisation de [MeAla]3-[EtVal]4-CsA permet d'obtenir une diminution de la taille de l'infarctus dans la zone à risque de l'ordre de 50% (Tableau 2). De plus, il a été relevé une diminution du taux de mortalité à 4 semaines (Fig. 1) ainsi qu'une meilleure fonction myocardique exprimée par une augmentation de la fraction d'éjection (Tableau 3).

Il est trouvé de manière surprenante que ces effets bénéfiques pour une même dose de composé actif ne sont pas obtenus lorsque l'administration du undécapeptide de l'invention est effectuée trop prématurément ou trop tardivement par rapport à la reperfusion.

### Exemple 3: Modèle de choc septique chez la souris - Influence de [MeAla]3-[EtVal]4-CsA sur la survie des souris septicémiques avec un dysfonctionnement myocardique.

Après anesthésie des souris, une péritonite par ligature et ponction cæcale est induite comme suit: réalisation d'une laparotomie médiane, extériorisation du caecum et ponction unique de celui-ci au dessous de la valve iléocaecale avec un aiguille de 21-gauge. Le caecum est replacé dans la cavité abdominale qui est ensuite suturée en 2 plans. Après réveil, les souris reçoivent par injection parentérale de l'antalgique nalbuphine.

Un groupe d'animaux reçoit du sérum physiologique (groupe contrôle); un autre groupe est traité par [MeAla]3-[EtVal]4-CsA à raison de 10 mg/kg par injection parentérale juste après induction chirurgicale du choc septique. Les animaux sont suivis pendant 4 jours et la mortalité rapportée pour chaque groupe.

La Fig. 2 représente l'amélioration de la survie des souris septicémiques traitées avec [MeAla]³-[EtVal]⁴-CsA par rapport aux animaux du groupe contrôle. 72 heures après induction du sepsis et début du traitement, le taux de survie pour les animaux traités est d'environ 60% par rapport aux animaux du groupe contrôle dont 100% de mortalité est atteinte en 36 heures. Cet effet s'explique essentiellement par l'effet cardioprotecteur de [MeAla]3-[EtVal]4-CsA sur le dysfonctionnement myocardique.

## Revendications

1. Undécapeptide cyclique ayant la structure de formule (I) 
-MeBmt-αAbu-MeAla-EtVal-Val-MeLeu-Ala-(D)Ala-MeLeu-MeLeu-MeVal-
1 2 3 4 5 6 7 8 9 10 11
Formule (I)
 pour utilisation comme médicament lors d'une situation d'ischémie myocardique nécessitant le rétablissement du flux sanguin et devant être administré préalablement et/ou pendant le rétablissement dudit flux sanguin.

2. Undécapeptide cyclique pour utilisation selon la revendication 1 lorsque ladite situation se manifeste par un dysfonctionnement cardiaque.

3. Undécapeptide cyclique pour utilisation selon la revendication 2 lorsque ledit dysfonctionnement cardiaque est la conséquence de lésions cellulaires cardiaques.

4. Undécapeptide cyclique pour utilisation selon l'une quelconque des revendications précédentes lorsque ladite situation d'ischémie myocardique se rencontre parmi le groupe des maladies suivantes: l'infarctus aigu du myocarde, l'angine de poitrine, l'angor instable, les maladies athéromateuses avec thromboembolie, les vasospasmes, les anévrysmes des petites et moyennes artères et des grands vaisseaux, l'hypotension artérielle due à une maladie du coeur ou systémique, incluant des infections graves tel un sepsis sévère avec ou sans choc septique ou des réactions allergiques, une hypotension due à l'effet de un ou plusieurs médicaments, drogues, poisons, produits toxiques.

5. Undécapeptide cyclique pour utilisation selon l'une quelconque des revendications précédentes lorsque ledit flux sanguin est rétabli par une reperfusion obtenue mécaniquement par angioplastie coronaire et/ou médicalement à la suite d'un traitement de fibrinolyse et/ou à la suite de mesures de réanimation médicamenteuse.

6. Undécapeptide cyclique pour utilisation selon la revendication 5 lorsque le début de l'administration dudit undécapeptide cyclique de formule (I) s'échelonne au cours d'une période allant de la trentième minute précédant la reperfusion jusqu'au moment de la réouverture de l'artère et/ou depuis le rétablissement du flux sanguin.

7. Undécapeptide cyclique pour utilisation selon la revendication 6 lorsque le début de l'administration dudit undécapeptide cyclique de formule (I) se passe dans période allant au plus tôt lors de la déclaration du sepsis et au plus tard 72 heures.

8. Undécapeptide cyclique pour utilisation selon l'une quelconque des revendications précédentes lorsque ledit undécapeptide cyclique de formule (I) est administré à une dose allant de 0,1 mg/kg à 30 mg/kg.

9. Undécapeptide cyclique selon l'une quelconque des revendications précédentes lorsque ledit undécapeptide cyclique de formule (I) est co-administré ou administré séparément avec au moins un second composé actif dans le traitement anti-angineux et/ou anti-septique.

10. Undécapeptide cyclique pour utilisation selon l'une quelconque des revendications précédentes lorsque ledit undécapeptide cyclique de formule (I) est administré par voie intraveineuse ou intra-artérielle ou intra-coronarienne.

## Claims

1. Cyclic undecapeptide having the structure of formula (I) 
-MeBmt-αAbu-MeAla-EtVal-Val-MeLeu-Ala-(D)Ala-MeLeu-MeLeu-MeVal-
1 2 3 4 5 6 7 8 9 10 11
Formula (I)
 for use as a medicament during myocardial ischemic event requiring the reestablishment of blood flow and which must be administered prior to or during the reestablishment of said blood flow.

2. Cyclic undecapeptide for use according to Claim 1, when said event manifests itself in terms of a cardiac dysfunction.

3. Cyclic undecapeptide for use according to Claim 2, when said cardiac dysfunction is the consequence of cardiac cell lesions.

4. Cyclic undecapeptide for use according to any one of the preceding claims, when said myocardial ischemic event is encountered among the group of the following diseases: acute myocardial infarction, angina pectoris, unstable angina, atheromatous diseases with thromboembolism, vasospasms, aneurisms of the small and medium arteries and of the large vessels, arterial hypotension due to a heart or systemic disease, including serious infections such as a severe sepsis with or without septic shock or allergic reactions, and hypotension due to the effect of one or more medicaments, drugs, poisons or toxic products.

5. Cyclic undecapeptide for use according to any one of the preceding claims, when said blood flow is reestablished by means of a reperfusion obtained mechanically by coronary angioplasty and/or medically following a fibrinolysis treatment and/or following drug-based reanimation steps.

6. Cyclic undecapeptide for use according to Claim 5, when the beginning of the administration of said cyclic undecapeptide of formula (I) is spread over a period ranging from the thirtieth minute preceding the reperfusion up to the moment when the artery is reopened and/or from the reestablishment of blood flow.

7. Cyclic undecapeptide for use according to Claim 6, when the beginning of the administration of said cyclic undecapeptide of formula (I) takes place in a period from, at the earliest, when the sepsis sets in and, at the latest, 72 hours.

8. Cyclic undecapeptide for use according to any one of the preceding claims, when said cyclic undecapeptide of formula (I) is administered at a dose ranging from 0.1 mg/kg to 30 mg/kg.

9. Cyclic undecapeptide for use according to any one of the preceding claims, when said cyclic undecapeptide of formula (I) is coadministered with or administered separately from at least a second compound that is active in anti-angina and/or anti-sepsis treatment.

10. Cyclic undecapeptide for use according to any one of the preceding claims, when said cyclic undecapeptide of formula (I) is administered by intravenous, intraarterial or intracoronary injection.

## Patentansprüche

1. Cyclisches Undecapeptid mit der Struktur der Formel (I) 
-MeBmt-αAbu-MeAla-EtVal-Val-MeLeu-Ala-(D)Ala-MeLeu-MeLeu-MeVal-
1 2 3 4 5 6 7 8 9 10 11
Formel (I)
 zur Verwendung als Medikament bei einer myokardischämischen Situation, die die Wiederherstellung des Blutflusses erfordert, das vor und/oder während der Wiederherstellung des Blutflusses zu verabreichen ist.

2. Cyclisches Undecapeptid zur Verwendung nach Anspruch 1, wobei sich die Situation durch eine Herzdysfunktion manifestiert.

3. Cyclisches Undecapeptid zur Verwendung nach Anspruch 2, wobei die Herzdysfunktion auf Herzzellenläsionen zurückzuführen ist.

4. Cyclisches Undecapeptid zur Verwendung nach einem der vorhergehenden Ansprüche, wobei die myokardischämische Situation aus der Gruppe der folgenden Erkrankungen stammt: akuter Myokardinfarkt, Angina pectoris, instabile Angina, atheromatöse Erkrankungen mit Thromboembolie, Vasospasmen, Aneurysmen der kleinen und mittleren Arterien und der großen Gefäße, arterielle Hypotonie infolge einer Herzerkrankung oder systemischen Erkrankung, u.a. schweren Infektionen wie einer schweren Sepsis mit oder ohne septischen Schock oder allergischen Reaktionen, Hypotonie infolge der Wirkung eines oder mehrerer Medikamente, Drogen, Gifte oder toxischer Produkte.

5. Cyclisches Undecapeptid zur Verwendung nach einem der vorhergehenden Ansprüche, wobei die Wiederherstellung des Blutflusses durch eine mechanisch durch Koronarangioplastie und/oder medizinisch nach Fibrinolysebehandlung und/oder nach medikamentösen Wiederbelebungsmaßnahmen erhaltene Reperfusion erfolgt.

6. Cyclisches Undecapeptid zur Verwendung nach Anspruch 5, wobei der Beginn der Verabreichung des cyclischen Undecapeptids der Formel (I) sich über einen Zeitraum von der dreißigsten Minute vor der Reperfusion bis zum Moment der Wiederöffnung der Arterie und/oder ab der Wiederherstellung des Blutflusses erstreckt.

7. Cyclisches Undecapeptid zur Verwendung nach Anspruch 6, wobei der Beginn der Verabreichung des cyclischen Undecapeptids der Formel (I) in einem Zeitraum von frühestens bei der Feststellung der Sepsis und spätestens 72 Stunden erfolgt.

8. Cyclisches Undecapeptid zur Verwendung nach einem der vorhergehenden Ansprüche, wobei das cyclische Undecapeptid der Formel (I) in einer Dosis von 0,1 mg/kg bis 30 mg/kg verabreicht wird.

9. Cyclisches Undecapeptid zur Verwendung nach einem der vorhergehenden Ansprüche, wobei das cyclische Undecapeptid der Formel (I) zusammen oder separat mit mindestens einer zweiten Verbindung, die bei der Antiangina-und/oder Antisepsis-Behandlung wirksam ist, verabreicht wird.

10. Cyclisches Undecapeptid zur Verwendung nach einem der vorhergehenden Ansprüche, wobei das cyclische Undecapeptid der Formel (I) auf intravenösem oder intraarteriellem oder intrakoronarem Weg verabreicht wird.
